# EUROPEAN PATENT SPECIFICATION

(11) **EP 3 332 779 B1**
(45) Date of publication and mention of the grant of the patent: **28.10.2020**
(21) Application number: 16833066.0
(22) Date of filing: 03.08.2016
(51) Int. Cl.: A61K 31/122, A61P 13/10

(54) **AMELIORATING AGENT FOR DETRUSOR HYPERACTIVITY WITH IMPAIRED CONTRACTILITY**
LINDERUNGSMITTEL FÜR DETRUSORHYPERAKTIVITÄT MIT BEEINTRÄCHTIGTER KONTRAKTILITÄT
AGENT VISANT À REMÉDIER À L'HYPERACTIVITÉ DU DÉTRUSOR AVEC ALTÉRATION DE LA CONTRACTILITÉ

(30) Priority: 04.08.2015 US 201514817318
(43) Date of publication of application: 13.06.2018
(73) Proprietor: Taiho Pharmaceutical Co., Ltd., Chiyoda-ku Tokyo 101-8444 (JP)
(72) Inventor: HAYASHI, Yukio, Tsukuba-shi Ibaraki 300-2611 (JP); NOMA, Takahisa, Tsukuba-shi Ibaraki 300-2611 (JP)
(74) Representative: Hoffmann Eitle
(86) International application number: PCT/JP2016/072753
(87) International publication number: WO 2017/022787

(56) References cited:
- EP-A1- 2 853 261
- WO-A1-02/066024
- WO-A1-2009/151035
- JP-A- 2002 241 271
- JP-A- 2015 067 565
- JP-A- 2015 086 212
- SAITO M ET AL: "Effect of cyclohexenonic long-chain fatty alcohol on rat overactive bladder induced by bladder neck obstruction", EUROPEAN JOURNAL OF PHARMACO, ELSEVIER SCIENCE, NL, vol. 501, no. 1-3, 6 October 2004 (2004-10-06), pages 143-149, XP004587604, ISSN: 0014-2999, DOI: 10.1016/J.EJPHAR.2004.08.005
- NOMIYA MASANORI ET AL: "MP8-12 A NEUROTROPHIC AGENT, N-HEXACOSANOL, PREVENTS THE DEVELOPMENT OF BLADDER HYPERACTIVITY IN A RAT MODEL OF CHRONIC BLADDER ISCHEMIA", JOURNAL OF UROLOGY, vol. 193, no. 4, 15 May 2015 (2015-05-15), XP029153350, ISSN: 0022-5347, DOI: 10.1016/J.JURO.2015.02.279
- Takeshi Watanabe ET AL: "Effects of long-chain fatty alcohol on peripheral nerve conduction and bladder function in diabetic rats", Life Sciences, 1 January 2002 (2002-01-01), pages 2215-2224, XP055362530, Retrieved from the Internet: URL:http://ac.els-cdn.com/S002432050101536 3/1-s2.0-S0024320501015363-main.pdf?_tid=4 b3ca132-1abf-11e7-bdf1-00000aab0f6c&acdnat =1491479636_3cae3988a77c2f04de4c1ee9d2401a 98
- SAITO, M. ET AL.: 'Effect of cyclohexenonic long-chain fatty alcohol on rat overactive bladder induced by bladder neck obstruction' EUR. J. PHARMACOL. vol. 501, 2004, pages 143 - 149, XP055361861
- SHUKO KOKUBUN ET AL.: 'Heisoku Boko ni Okeru Joshinkei to Boko Heikatsukin no Shushuku Shogai -Cyclohexenones Yudotai(TAC-302) no Joshinkei Yobo Koka' THE JAPANESE JOURNAL OF UROLOGY vol. 102, no. 2, 2011, page 352, XP009509045
- NOMIYA, M. ET AL.: 'A neurotrophic agent, N- hexacosanol, prevents the development of bladder hyperactivity in a rat model of chronic bladder ischemia' J. UROLOGY vol. 193, no. 4S, April 2015, page E77, XP029153350
- WATANABE, T. ET AL.: 'Effects of long- chain fatty alcohol on peripheral nerve conduction and bladder function in diabetic rats' LIFE SCIENCES vol. 70, 2002, pages 2215 - 2224, XP055362530
- TATEMICHI SATOSHI ET AL: "Effects of Silodosin, an alpha(1A)-Adrenoceptor Antagonist, and Distigmine, an Acetylcholinesterase Inhibitor, and Their Combined Effects on Impaired Voiding Function in Zucker Diabetic Fatty Rats", PHARMACOLOGY (BASEL), vol. 95, no. 5-6, 2015, pages 285-292, ISSN: 0031-7012
- SAWADA NORIFUMI ET AL: "Protective Effect of a beta(3)-Adrenoceptor Agonist on Bladder Function in a Rat Model of Chronic Bladder Ischemia", EUROPEAN UROLOGY, vol. 64, no. 4, October 2013 (2013-10), pages 664-671, ISSN: 0302-2838

## Description

### Technical Field:

The present invention relates to 3-(15-hydroxypentadecyl)-2,4,4-trimethyl-2-cyclohexene-1-one, a salt thereof, or a solvate thereof, as an active component, for use in the treatment of detrusor hyperactivity with impaired contractility (DHIC), excluding disorders based on vesicourethral dyssynergia, as defined in the claims.

### Background Art:

3-(15-hydroxypentadecyl)-2,4,4-trimethyl-2-cyclohexene-1-one (hereinafter referred to as "Compound 1 of the present invention") is a compound having a structure represented by Formula (1) below.

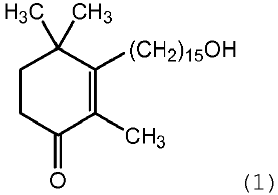

Patent Document 1 discloses that a cyclohexenone long-chain alcohol comprising the compound represented by Formula (1) has an effect of promoting neurite growth, and thus is useful as a preventive and/or therapeutic agent for brain disorders such as dementia. Patent Document 2 discloses that a cyclohexenone long-chain alcohol comprising the compound represented by Formula (1) is useful as a therapeutic agent for treating dysuria. Patent Document 3 discloses that the compound represented by Formula (1) is useful as a therapeutic agent for diseases based on vesicourethral dyssynergia.

However, the effect as a therapeutic agent for treating dysuria shown in Patent Document 2 was confirmed only against dysuria with depressed bladder function (the effect was confirmed by the improvement in maximum voided volume, bladder capacity, and micturition efficiency). More specifically, the effect of ameliorating detrusor hyperactivity with impaired contractility of Compound 1 of the present invention has been completely unknown.

Further, Patent Document 3 merely confirmed time-lag alleviation effects (Example 1) and accompanying maximum voided volume alleviation effects (Example 2) using rat models presenting a time-lag between bladder detrusor contraction and urethral sphincter relaxation four weeks after induction of dysuria by Streptozotocin (STZ); Patent Document 3 nowhere discloses or suggests that Compound 1 of the present invention is effective for a disorder that presents both detrusor hyperactivity and impaired contractility.

In the first place, as shown in the later-described Reference Examples, the dysuria model disclosed in the Examples of Patent Document 3 is a model that does not present detrusor hyperactivity; that is, this model cannot be used for the evaluation of therapeutic effects for a disorder that presents both detrusor hyperactivity and impaired contractility.

Patent Document 3 discloses in paragraph [0032] many diseases as diseases based on vesicourethral dyssynergia, and discloses "detrusor hyperreflexia with impaired contractile function (DHIC)" as one of these diseases. However, this is merely an example of diseases that may be newly developed because of vesicourethral dyssynergia. Further, as is clear from the description of paragraph [0033] stating that diseases that are not accompanied by vesicourethral dyssynergia fall out of the range of diseases treatable by the invention, a disorder that presents both detrusor hyperactivity and impaired contractility, in particular, a disorder that presents both detrusor hyperactivity and impaired contractility caused by vesicourethral dyssynergia, is not the target of the invention of Patent Document 3.

In normal micturition function, detrusor does not contract but relaxes during the storage phase (urine can be reserved in the bladder), and contracts during only the voiding phase. In storage dysfunction disease (overactive bladder), detrusor overactivity occurs during the storage phase, so urine cannot be reserved fully in the bladder. Medicines, such as an anti-cholinergic agent and a β3 receptor agonist, are effective for a storage dysfunction disease. On the other hand, a urine-voiding dysfunction disease (underactive bladder) induces detrusor impaired contractility and causes problematic increased residual urine volume. Medicines, such as a cholinesterase inhibitor and a cholinergic agonist, are used for a urine-voiding dysfunction disease. However, it is generally known that an effective agent for overactive bladder does not work or is even detrimental against an underactive bladder (Non-patent Document 1, Non-patent Document 2). Also, it is known that an effective agent for an underactive bladder is invalid or detrimental against an overactive bladder (Non-patent Document 3). In these ways, although there are some therapeutic approaches against an individual disease of an overactive bladder or underactive bladder, almost no agent is expected to be effective against a disease condition in which both an overactive bladder and an underactive bladder are present. Therefore, there is no means for treating such a condition.

DHIC is a disorder which presents both detrusor overactivity and detrusor impaired contractility in the body of the same individual (Non-patent Document 4, Non-patent Document 5). In recent years, detrusor hyperactivity with impaired contractility has been recognized as a new disease different from an overactive bladder or underactive bladder. A large number of patients having detrusor hyperactivity with impaired contractility have been reported clinically, and it is known that about 20 percent of those 65 years old or older suffer from detrusor hyperactivity with impaired contractility (Non-patent Document 6). Based on demographic information, the assumed number of patients having detrusor hyperactivity with impaired contractility in 2025 will be about 4.4 million in Japan. Because an increase in residual urine volume due to detrusor impaired contractility additionally occurs with detrusor hyperactivity, it induces high-pressure during the storage phase and incontinence. Further, if such a condition is left unattended without appropriate care, it results in a severe disease, such as urinary-tract infection, upper urinary tract disorder, or renal dysfunction.

DHIC is clinically diagnosed by confirming the coexistence of detrusor overactivity during the storage phase and detrusor impaired contractility during the voiding phase using a Pressure-Flow Study (a nomogram analysis is useful for the diagnosis) (Non-patent Document 4, Non-patent Document 5).

Also, because DHIC is a disorder which presents both detrusor overactivity and detrusor impaired contractility, the diagnosis of an overactive bladder and an underactive bladder can be available for the diagnosis of DHIC (Non-patent Document 7, Non-patent Document 8). Those are, an overactive bladder is diagnosed by subjective symptoms (urgency, incontinence, pollakiuria etc.), and an underactive bladder is diagnosed by subjective symptoms (forceless urinary stream, terminal dribbling, retarded micturition, abdominal straining to urinate, a feeling of residual urine, urinary retention, etc.), uroflowmetry, measurement of residual urine volume, etc. Then, DHIC can be diagnosed by confirming the coexistence of both the disorders.

In therapeutic strategy for DHIC, because conflicting dysfunctions of detrusor overactivity and detrusor impaired contractility coexist in the body of the same individual, it is therapeutic high-difficult dysuria. As described above, generally, the ameliorating agent for treating either an overactive bladder or an underactive bladder is not only clinically insufficient for patients with DHIC but also has a risk of exacerbation of the symptoms. Considering this situation, we need a therapeutic agent for DHIC which presents both detrusor overactivity and detrusor impaired contractility in the body of the same individual. It was recently reported that an α1-blocker, such as Tamsulosin, etc., which was effective for urine-voiding dysfunction (underactive bladder), was also effective for an overactive bladder (Non-patent Document 9).

European Journal of Pharmacology 501 (2004) 143-149 describes the investigation of the effect of cyclohexenonic long-chain fatty alcohol on rat overactive bladder induced by bladder neck obstruction.

EP 2 853 261 A1 relates to an ameliorating agent for a disease based on vesicourethral dyssynergia, comprising, as an active ingredient, 3-(15-hydroxypentadecyl)-2,4,4-trimethyl-2-cyclohexene-1-one, a salt thereof, or a solvate thereof. The disease based on vesicourethral dyssynergia is any of dysuria accompanying a lifestyle-related disease (such as diabetic dysuria), idiopathic dysuria, dysuria after pelvic surgery, dysuria accompanying spinal cord injury, dysuria accompanying spinal canal stenosis, dysuria accompanying benign prostatic hypertrophy, dysuria accompanying high-pressure voiding/high-pressure urine storage, neurogenic or non-neurogenic lower urinary tract symptoms (LUTS), detrusor sphincter dyssynergia, and detrusor bladder neck dyssynergia.

The Journal of Urology Vol. 193, No. 4S, Supplement, 2015, e77, describes a study using a rat model of chronic bladder ischemia wherein it was investigated whether the prophylactic administration of N-hexacosanol can prevent the development of detrusor overactivity.

Life Sciences 70 (2002) 2215-2224 relates to the effects of long-chain fatty alcohol on peripheral nerve conduction and bladder function in diabetic rats.

### Citation List:

### Patent Documents:

Patent Document 1: International Publication WO1999/008987
Patent Document 2: International Publication WO2002/066024
Patent Document 3: International Publication WO2015/046377

### Non-patent Documents:

Non-patent Document 1: J Smooth muscle Res 48, p115-124 (2012)
Non-patent Document 2: Br J Urol 82, p272-277 (1998)
Non-patent Document 3: J Urol 174, p1137-1141 (2005)
Non-patent Document 4: JAMA, 257, p3076-3081 (1987)
Non-patent Document 5: Rev Hosp Clin Fac Med Sao Paulo, 59, p206-215 (2004)
Non-patent Document 6: Hainyo-Shogai Practice [Voiding disorders digest], 14, p299-306 (2007)
Non-patent Document 7: Neurourol Urodyn, 29, p4-20 (2010)
Non-patent Document 8: Eur Urol., 65, p389-398 (2014)
Non-patent Document 9: JAMA 296, p2319-2328 (2006)

### Summary of Invention:

### Technical Problem:

An object of the present invention is to provide a method for treating DHIC by improving both detrusor overactivity and detrusor impaired contractility.

### Solution to Problem:

The inventors of the present invention carried out extensive research to attain the above object, and found that 3-(15-hydroxypentadecyl)-2,4,4-trimethyl-2-cyclohexene-1-one represented by Formula (1) below ameliorates both detrusor overactivity and detrusor impaired contractility, and thus is useful as a therapeutic agent for treating a disorder based on DHIC.

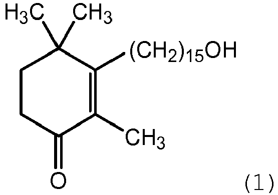

More specifically, the present invention encompasses the following items.

Item 1. A therapeutic agent comprising 3-(15-hydroxypentadecyl)-2,4,4-trimethyl-2-cyclohexene-1-one, a salt thereof, or a solvate thereof for use in the treatment of detrusor hyperactivity with impaired contractility, excluding disorders based on vesicourethral dyssynergia.

Item 2. A pharmaceutical composition comprising 3-(15-hydroxypentadecyl)-2,4,4-trimethyl-2-cyclohexene-1-one, a salt thereof, or a solvate thereof, and a pharmaceutical carrier for use in the treatment of detrusor hyperactivity with impaired contractility, excluding disorders based on vesicourethral dyssynergia.

Item 3. 3-(15-hydroxypentadecyl)-2,4,4-trimethyl-2-cyclohexene-1-one, a salt thereof, or a solvate thereof for use in the treatment of detrusor hyperactivity with impaired contractility, excluding disorders based on vesicourethral dyssynergia.

### Advantageous Effects of Invention:

The present invention enables effective treatments of DHIC.

### Brief Description of Drawings:

Fig. 1 shows representative cystometry charts of a rat dysuria model that presents DHIC.
Fig. 2 shows the effects of Compound 1 of the present invention and α1-blocker (Tamsulosin) on detrusor contractility in a rat dysuria model that presents DHIC.
   Sham: n=11, Control (6% Gelucire): n=19, Compound 1 of the present invention (10 mg/kg × 2/day p.o.): n=8, Tamsulosin (0.3 mg/kg, i.v.): n=6
Fig. 3 shows representative cystometry charts of a rat dysuria model that presents vesicourethral dyssynergia.

### Description of Embodiments:

Compound 1 of the present invention is a known compound, and is produced by, for example, the method disclosed in International Publication WO1999/008987.

The "disorder that presents both detrusor hyperactivity and detrusor impaired contractility" of the present invention means a disease condition that presents both detrusor overactivity during the urine storage phase and detrusor impaired contractility during the voiding phase; specifically, it means detrusor hyperactivity with impaired contractility (DHIC).

Detrusor hyperactivity with impaired contractility may be diagnosed by confirming detrusor overactivity during the urine storage phase and detrusor impaired contractility during the voiding phase using a Pressure-Flow Study (a nomogram analysis is useful) (JAMA, 257, p3076-3081 (1987); Rev Hosp Clin Fac Med Sao Paulo, 59, p206-215 (2004)).

Also, because detrusor hyperactivity with impaired contractility is a disorder which presents both detrusor overactivity and detrusor impaired contractility, methods for diagnosing an overactive bladder and an underactive bladder can be used for the diagnosis of detrusor hyperactivity with impaired contractility (Neurourol Urodyn, 29, p4-20 (2010); Eur Urol, 65, p389-398 (2014)). More specifically, an overactive bladder is diagnosed by subjective symptoms (urination urgency, incontinence, pollakiuria, etc.), and an underactive bladder is diagnosed by subjective symptoms (forceless urinary stream, terminal dribbling, retarded micturition, abdominal straining to urinate, a feeling of residual urine, urinary retention, etc.) as well as uroflowmetry, measurement of residual urine volume, and the like. Then, DHIC can be diagnosed by confirming the coexistence of both of the disorders.

In the present invention, the cause of detrusor hyperactivity with impaired contractility (DHIC) is not limited insofar as the disease is a condition with a coexistence of detrusor overactivity during the urine storage phase and detrusor impaired contractility during the voiding phase; and insofar as detrusor hyperactivity with impaired contractility (DHIC) that is based on or caused by vesicourethral dyssynergia is excluded.

The "method for treating a disorder that presents both detrusor hyperactivity and detrusor impaired contractility" of the present invention encompasses a method for treating or ameliorating a disorder that presents both detrusor hyperactivity and detrusor impaired contractility, a method for ameliorating the symptoms of the disorder, and a maintenance treatment for preventing the recurrence of the disorder.

The "method for treating detrusor hyperactivity with impaired contractility" of the present disclosure includes a method for treating or ameliorating detrusor hyperactivity with impaired contractility, a method for ameliorating the symptoms of the disorder, and a maintenance treatment for preventing the recurrence of the disorder.

Examples of the "symptoms" of the disorder that presents both detrusor hyperactivity and detrusor impaired contractility, and the "symptoms" of detrusor hyperactivity with impaired contractility include subjective symptoms such as urination urgency, incontinence, and pollakiuria, forceless urinary stream, terminal dribbling, retarded micturition, abdominal straining to urinate, a feeling of residual urine, urinary retention, and the like, as well as decrease in urine flow, and increase in residual urine volume. In particular, Compound 1 of the present invention is capable of simultaneously ameliorating any one of urination urgency, incontinence, and pollakiuria, which are symptoms of detrusor hyperactivity, and any one of forceless urinary stream, terminal dribbling, retarded micturition, abdominal straining to urinate, a feeling of residual urine, urinary retention, decrease in urine flow, and increase in residual urine volume, which are symptoms of detrusor impaired contractility.

Compound 1 of the present invention may form acid adduct salt, or base adduct salt. And the present invention includes a salt thereof to the extent that the salt is a pharmaceutically acceptable salt thereof. Specifically, it includes an acid adduct salt with an inorganic acid, such as hydrochloric acid, hydrobromic acid, hydroiodic acid, sulfuric acid, nitric acid, or phosphoric acid, etc.; an acid adduct salt with an organic acid such as formic acid, acetic acid, propionic acid, oxalic acid, malonic acid, succinic acid, fumaric acid, maleic acid, lactic acid, malic acid, citric acid, tartaric acid, carbonic acid, picric acid, methansulfonic acid, p-toluenesulfonic acid, or glutamic acid, etc.; salt with an inorganic base, such as sodium, potassium, magnesium, calcium, or aluminum, etc.; salt with an organic base, such as methylamine, ethylamine, meglumine, or ethanolamine, etc.; and salt with basic amino acid, such as lysine, arginine, or ornithine; ammonium salt, etc.

Examples of the solvent of the solvate of Compound 1 of the present invention include water, methanol, ethanol, isopropanol, acetonitrile, tetrahydrofuran, ethyl acetate, toluene, hexane, acetone, methyl ethyl ketone, methyl isobutyl ketone, etc.

3-(15-hydroxypentadecyl)-2,4,4-trimethyl-2-cyclohexene-1-one, a salt thereof, or a solvate thereof of the present invention can be prepared into various dosage forms by using known preparation methods using a pharmaceutically acceptable carrier. The dosage form is not particularly limited, and examples thereof include oral agents, such as tablets, coated tablets, pills, powdered drugs, granules, capsules, liquids, suspensions, or emulsions; and parenteral agents, such as injections or suppositories.

In preparing tablets, examples of carriers include excipients, such as lactose, sucrose, sodium chloride, glucose, urea, starch, calcium carbonate, kaolin, crystalline cellulose, or silicic acid; binders, such as water, ethanol, propanol, cornstarch, simple syrup, glucose solution, starch solution, gelatin solution, carboxymethylcellulose, shellac, methylcellulose, hydroxypropylcellulose, hydroxypropylmethylcellulose, potassium phosphate, or polyvinyl pyrrolidone; disintegrants, such as dry starch, sodium alginate, powdered agar, powdered laminaran, sodium hydrogencarbonate, calcium carbonate, polyoxyethylene sorbitan fatty acid ester, sodium lauryl sulfate, stearic acid monoglyceride, or lactose; disintegration inhibitors, such as sucrose, stearic acid, cacao butter, or hydrogenated oils; absorbefacients, such as quaternary ammonium salts or sodium lauryl sulfate; moisturizers, such as glycerin or starch; adsorbents, such as starch, lactose, kaolin, bentonite, or colloidal silicic acid; and lubricants, such as purified talc, stearate, boric acid powder, or polyethylene glycol. Further, the tablets may be generally coated tablets, such as sugar-coated tables, gelatin-coated tablets, enteric-coated tablets, film-coated tablets, double-coated tablets, or multi-coated tablets.

In preparing pills, examples of the carrier, include excipients, such as glucose, lactose, starch, cacao butter, hardened vegetable oil, kaolin, or talc; binders, such as gum arabic powder, tragacanth powder, gelatin, or ethanol; and disintegrators, such as laminaran or agar. Capsules are usually prepared in a standard method by blending the drug with one or more carriers as exemplified above, and encapsulating the mixture into hard gelatin capsules, soft capsules, etc.

In preparing oral liquid formulations, an internal liquid medicine, a syrup, an elixir, or the like, may be prepared by a standard method using a sweetening/flavoring agent, buffer, stabilizer, etc. In this case, examples of sweetening/flavoring agents include sucrose, wild orange peel, citric acid, and tartaric acid; examples of buffers include sodium citrate; and examples of stabilizers include tragacanth, gum arabic, and gelatin.

In preparing suppositories, examples of usable carriers include polyethylene glycol, cacao butter, higher alcohols, esters of higher alcohols, gelatin, and semisynthetic glycerides.

In preparing injections, the liquids, emulsions, and suspensions are preferably sterilized and rendered isotonic to the blood. Examples of diluents for preparing such dosage forms include water, aqueous lactic acid solution, ethanol, propylene glycol, macrogols, ethoxylated isostearyl alcohol, polyoxyethylenated isostearyl alcohol, and polyoxyethylene sorbitan fatty acid ester.

In this case, sodium chloride, glucose, or glycerin in an amount sufficient to prepare an isotonic solution may be added to the pharmaceutical formulation. Further, general solubilizers, buffers, anesthetics, and the like, may also be added to the pharmaceutical formulation. Additionally, coloring agents, preservatives, aromatics, flavors, sweetening agents, or other medicinal products may be incorporated, if necessary, into the pharmaceutical formulations.

The method for administering the DHIC ameliorating agent of the present invention is not particularly limited, and is suitably selected according to the dosage form thereof, the age, gender, and other conditions of the patient, the severity of the symptoms of the patient, and the like. For example, tablets, pills, powdered drugs, granules, capsules, liquids, suspensions, and emulsions are orally administered. The injections are intravenously administered singly, or as a mixture with a general infusion liquid, such as liquid glucose or an amino acid liquid. Further, as necessary, the injections are singly administered intra-arterially, intramuscularly, intradermally, subcutaneously, or intraperitoneally. The suppositories are intrarectally administered.

The amount of the compound of the present invention or a salt thereof to be incorporated into each of the above dosage unit form depends on the symptoms of the target patient, or depends on the drug form; however, the amount per dosage unit form is generally preferably about 0.005 to 1,000 mg, more preferably 1 to 800 mg, further preferably 5 to 500 mg for oral agents; about 0.001 to 500 mg, more preferably 0.02 to 400 mg, further preferably 1 to 250 mg for injections; and about 0.01 to 1,000 mg, more preferably 1 to 800 mg, and further preferably 5 to 500 mg for suppositories. Additionally, the daily dose for an adult of the drug to be administered with the above dosage form is generally about 0.005 to 5,000 mg, preferably 0.01 to 2,000 mg, more preferably 10 to 1600 mg, and further preferably 20 to 800 mg, although such doses depend on the symptoms, body weight, age, gender, etc., of the patient. For each day, the daily dose is preferably taken at one time, or divided into two to four administrations.

The present invention is more specifically described below in reference to the Test Examples; however, the present invention is not limited to these examples.

### Examples:

### Test Example 1:

### Preparation of a rat model that presents DHIC

The models were produced by partial ligation (φ1.57 mm) of the urethra in rats (9 weeks, female, Sprague-Dawley). Six weeks after preparation of the model, the rats were released from the ligation. The next day, the intravesical pressure and the voided volume were measured under an awake condition. And the detrusor contractility during the voiding phase was evaluated by nomogram analysis using Qmax (maximum urine flow rate) and Pdet (detrusor pressure). Additionally, the detrusor overactivity, as an index of an overactive bladder, and the increase of residual urine volume, as an index of an underactive bladder, were evaluated.

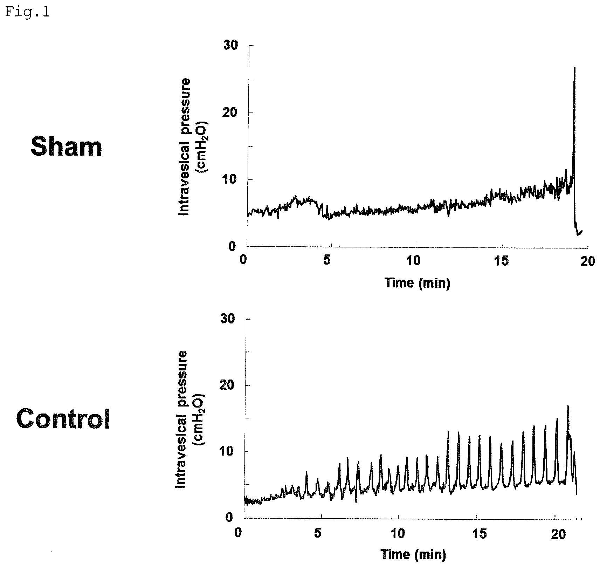

Fig. 1 shows representative cystometry charts. In the dysuria model rat (control) compared to the sham rat, there are characteristics of detrusor hyperactivity with impaired contractility that are characterized by remarkable overactivity and increased residual urine volume (Table 1).

**Table 1**

| The effects of Compound 1 of the present invention and α1-blocker (Tamsulosin) on detrusor overactivity and residual urine volume in a rat dysuria model that presents DHIC. | | | |
|---|---|---|---|
| Group | N | Detrusor overactivity (times/min) | Residual urine volume (mL) |
| Sham | 11 | 0.19 ± 0.09 | 0.10 ± 0.03 |
| Control | 19 | 1.73 ± 0.10 # | 0.57 ± 0.06 # |
| Compound 1 of the present invention | 8 | 0.63 ± 0.10 * | 0.28 ± 0.13 * |
| Tamsulosin | 6 | 0.68 ± 0.33 § | 0.64 ± 0.08 |

| | | | |
|---|---|---|---|
| #: p<0.05 vs. Sham group (unpaired Student's t-test) *: p<0.05 vs. Control group (unpaired Student's t-test) §: p<0.05 vs. Control group (unpaired Student's t-test) | | | |

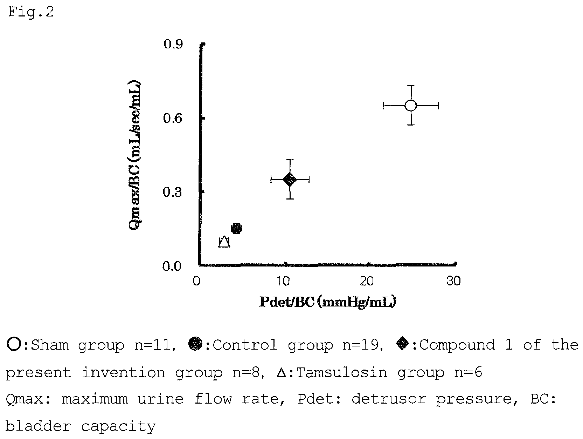

Through evaluation of detrusor contractility during the voiding phase in reference to a nomogram analysis which is used in clinical sites (Non-patent Document 9: Urol Clin North Am, 17, p553-566 (1990)), because the plot of the control group is positioned in a position relatively close to the origin compared to it of the sham group (the distance from the origin: Sham group 24.75±3.14, control group 4.24±0.53,p<0.05), it is judged that reduction of detrusor contractility occurs in the rat dysuria model (Fig.2).

In the rat dysuria model from these findings, it is confirmed that the detrusor overactivity during the storage phase and the reduction of detrusor contractility during the voiding phase in a nomogram analysis coexist in the body of the same individual which is a clinical diagnostic index of DHIC. It is found that the rat dysuria model is able to be evaluated as a model of DHIC.

### Test Example 2:

### Effects of ameliorating detrusor overactivity and detrusor impaired contractility in a rat dysuria model that presents DHIC.

The effect of 3-(15-hydroxypentadecyl)-2,4,4-trimethyl-2-cyclohexene-1-one (hereinafter referred to as "Compound 1 of the present invention") on DHIC was evaluated.

The dysuria models in this example were prepared in the same manner as in Test Example 1. The test drugs (vehicle: 6% Gelucire, Compound 1 of the present invention 10 mg/kg) were orally administered to each group after two weeks from the preparation of the models twice a day for four weeks. On the day of the final administration, the rats were released from the ligation of the urethra. The next day, the intravesical pressure and the voided volume were measured using cystometry under a conscious condition. The detrusor overactivity, as an index of an overactive bladder, and the increase of residual urine volume, as an index of an underactive bladder, were evaluated. Additionally, the detrusor contractility during the voiding phase was evaluated by a nomogram analysis using Qmax and Pdet.

In comparison with the detrusor overactivity (1.73 ± 0.10 times/min) and the residual urine volume (0.57 ± 0.06 mL) in a control group receiving vehicle (6% Gelucire), detrusor overactivity (0.63 ± 0.14 times/min) and residual urine volume (0.28 ± 0.13 mL) in the group receiving Compound 1 of the present invention were significantly improved (Table 1). Additionally, from the result of an evaluation using a nomogram analysis, because the plot of Compound 1 of the present invention group is in a position relatively distant from the origin compared to it of the control group (the distance from the origin: 10.5±2.3, p<0.05), it is judged that detrusor contractility is improved in Compound 1 of the present invention group compared to the control group (Fig. 2).

In a DHIC model in which detrusor overactivity and detrusor impaired contractility coexist, it is recognized that Compound 1 of the present invention possesses effects for ameliorating both detrusor overactivity and detrusor impaired contractility.

### Comparative Example 1:

### Effects of α1-blocker (Tamsulosin) on detrusor overactivity and detrusor underactivity/impaired contractility in a rat dysuria model that presents DHIC.

Effects of α1-blocker (Tamsulosin) widely used as a dysuria therapeutic agent with respect to detrusor overactivity with impaired contractility were examined.

In the same manner as Test Example 1, the rats were released from the ligation of the urethra at six weeks after the preparation of the model. The next day, the intravesical pressure and the voided volume were measured using cystometry under a conscious condition. The detrusor overactivity, as an index of an overactive bladder, and the increase of residual urine volume, as an index of an underactive bladder, were evaluated. Additionally, the detrusor contractility during the voiding phase was evaluated by a nomogram analysis using Qmax and Pdet. Tamsulosin (3 µg/kg) was administered intravenously to the dysuria rat at the evaluation (six weeks after preparation of the model).

Detrusor overactivity was significantly improved in the Tamsulosin (3 µg/kg) group (0.68 ± 0.33 times/min) compared to the control group (1.73 ± 0.10 times/min) (Table 1). However, Tamsulosin (3 µg/kg) had no effect on the residual urine volume (Table 1) and detrusor contractility in a nomogram analysis (Fig. 2) .

### Comparative Example 2:

### Effects of α1 blocker (Tamsulosin) on detrusor impaired contractility in a rat underactive bladder model

With reference to WO2013/027806, the effects of α1 blocker (Tamsulosin) on an underactive bladder were evaluated. The dysuria models in the present example were prepared by treating 10-week-old female Wistar rats with streptozotocin (65 mg/kg, i.p.). From four weeks after the preparation of the models, Tamsulosin (1 µg/kg/hr) was administered subcutaneously using an osmotic pump. Four weeks after the implant of the osmotic pump, the intravesical pressure and the voided volume were measured using cystometry under a urethane anesthesia condition. And the residual urine volume, as an index of an underactive bladder, was evaluated.

Table 2 shows the results. In comparison with the Sham group, a significant increase of the residual urine volume, which is an index of an underactive bladder, was observed in the control group (eight weeks after the development of the disease in the models). Tamsulosin showed a significant reduction in the increase of residual urine volume which was observed in the control group.

The above results suggest that Tamsulosin improves an underactive bladder, that is, detrusor impaired contractility.

**Table 2**

| Effects of α1 blocker (Tamsulosin) on residual urine volume in a rat underactive bladder model | | |
|---|---|---|
| Group | n | Residual urine volume (mL) |
| Control | 10 | 1.11 ± 0.20 |
| Tamsulosin | 9 | 0.47 ± 0.11 § |

| | | |
|---|---|---|
| §: p<0.05 vs. Control group (unpaired Student's t-test) | | |

### Reference Example: Preparation of a rat dysuria model showing vesicourethral dyssynergia

According to Test Example 1 of Patent Document 3, four weeks after the induction of dysuria by Streptozotocin (STZ), rat models presenting a time-lag between bladder detrusor contraction and urethral sphincter relaxation were prepared. Using this dysuria model, the bladder internal pressure was measured and the presence or absence of detrusor hyperactivity was evaluated under a urethane anesthesia condition.

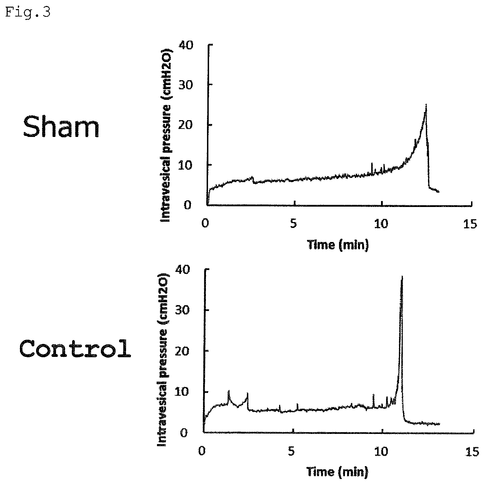

Fig. 3 shows representative cystometry charts. In a dysuria rat (control), the characteristic continuous peak of detrusor hyperactivity, which was observed in the dysuria rat (control) of Test Example 1, was not observed. It was thus revealed that the rat dysuria model presenting vesicourethral dyssynergia used in Test Example 1 of Patent Document 3 did not show the symptoms of detrusor hyperactivity, and that, therefore, this model was incapable of evaluating detrusor hyperactivity with impaired contractility.

Although an α1 blocker, which is generally used as a dysuria-treating drug, has an effect on an underactive bladder (Comparative Example 2) and is also reported as having an effect on improving an overactive bladder (Non-patent Document 10: J Urol, 190, p1116-1122 (2013)), the effect of Tamsulosin was not observed in dysuria (DHIC) in which detrusor overactivity and detrusor impaired contractility coexist in the body of the same individual (Comparative Example 1). On the other hand, Compound 1 of the present invention shows an effect on ameliorating both dysfunctions in DHIC in which detrusor overactivity and detrusor impaired contractility coexist (Test Example 2). Therefore, it is suggested that Compound 1 of the present invention is a useful therapeutic agent for DHIC (Test Example 2).

## Claims

1. A therapeutic agent comprising 3-(15-hydroxypentadecyl)-2,4,4-trimethyl-2-cyclohexene-1-one, a salt thereof, or a solvate thereof for use in the treatment of detrusor hyperactivity with impaired contractility, excluding disorders based on vesicourethral dyssynergia.

2. A pharmaceutical composition comprising 3-(15-hydroxypentadecyl)-2,4,4-trimethyl-2-cyclohexene-1-one, a salt thereof, or a solvate thereof, and a pharmaceutical carrier for use in the treatment of detrusor hyperactivity with impaired contractility, excluding disorders based on vesicourethral dyssynergia.

3. 3-(15-hydroxypentadecyl)-2,4,4-trimethyl-2-cyclohexene-1-one, a salt thereof, or a solvate thereof for use in the treatment of detrusor hyperactivity with impaired contractility, excluding disorders based on vesicourethral dyssynergia.

## Patentansprüche

1. Therapeutisches Mittel, umfassend 3-(15-Hydroxypentadecyl)-2,4,4-trimethyl-2-cyclohexen-1-on, ein Salz davon oder ein Solvat davon zur Verwendung bei der Behandlung von Detrusorhyperaktivität mit beeinträchtigter Kontraktilität, ausgenommen Störungen aufgrund von vesikoureteraler Dyssynergie.

2. Pharmazeutische Zusammensetzung, umfassend 3-(15-Hydroxypentadecyl)-2,4,4-trimethyl-2-cyclohexen-1-on, ein Salz davon oder ein Solvat davon und einen pharmazeutischen Träger zur Verwendung bei der Behandlung von Detrusorhyperaktivität mit beeinträchtigter Kontraktilität, ausgenommen Störungen aufgrund von vesikoureteraler Dyssynergie.

3. 3-(15-Hydroxypentadecyl)-2,4,4-trimethyl-2-cyclohexen-1-on, ein Salz davon oder ein Solvat davon zur Verwendung bei der Behandlung von Detrusorhyperaktivität mit beeinträchtigter Kontraktilität, ausgenommen Störungen aufgrund von vesikoureteraler Dyssynergie.

## Revendications

1. Agent thérapeutique comprenant du 3-(15-hydroxypentadécyl)-2,4,4-triméthyl-2-cyclohexène-1-one, un sel de celui-ci, ou un solvate de celui-ci destiné à être utilisé dans le traitement de l'hyperactivité du détrusor avec altération de la contractilité, en excluant des troubles basés sur une dyssynergie vésico-uréthrale.

2. Composition pharmaceutique comprenant du 3-(15-hydroxypentadécyl)-2,4,4-triméthyl-2-cyclohexène-1-one, un sel de celle-ci, ou un solvate de celle-ci, et un vecteur pharmaceutique destinés à être utilisés dans le traitement de l'hyperactivité du détrusor avec altération de la contractilité, en excluant des troubles basés sur une dyssynergie vésico-uréthrale.

3. 3-(15-hydroxypentadécyl)-2,4,4-triméthyl-2-cyclohexène-1-one, un sel de celui-ci, ou un solvate de celui-ci destiné à être utilisé dans le traitement de l'hyperactivité du détrusor avec altération de la contractilité, en excluant des troubles basés sur une dyssynergie vésico-uréthrale.
